# EUROPÄISCHE PATENTSCHRIFT

(11) **EP 1 182 258 B1**
(45) Veröffentlichungstag und Bekanntmachung des Hinweises auf die Patenterteilung: **22.02.2006**
(21) Anmeldenummer: 01115691.6
(22) Anmeldetag: 05.07.2001
(51) Int. Cl.: C12N 15/57, C12N 9/64, C12Q 1/68, C07K 16/40, A61K 38/48, G01N 33/573

(54) **Mutanten der den Faktor VII aktivierenden Protease und Nachweisverfahren mit spezifischen Antikörpern**
Mutants of the factor VII activating protease and methods for their detection
Mutants de la protéase pour l'activation du facteur de coagulation VII

(30) Priorität: 26.07.2000 DE 10036641; 10.10.2000 DE 10050040; 21.10.2000 DE 10052319; 12.04.2001 DE 10118706
(43) Veröffentlichungstag der Anmeldung: 27.02.2002
(73) Patentinhaber: ZLB Behring GmbH, 35041 Marburg (DE)
(72) Erfinder: Roemisch, Juergen, 35041 Marburg (DE); Stoehr, Hans-Arnold, 35083 Wetter (DE); Feussner, Annette, 35043 Marburg (DE); Lang, Wiegand, 35091 Cölbe (DE); Weimer, Thomas, 35075 Gladenbach (DE); Becker, Margret, 35043 Marburg (DE); Nerlich, Claudia, 35041 Marburg (DE); Muth-Naumann, Gudrun, 35083 Wetter (DE)

(56) Entgegenhaltungen:
- EP-A- 1 033 405
- EP-A- 1 059 359
- EP-A- 1 074 615
- DE-A- 19 903 693
- ROEMISCH J ET AL: "A PROTEASE ISOLATED FROM HUMAN PLASMA ACTIVATING FACTOR VII INDEPENDENT OF TISSUE FACTOR" BLOOD COAGULATION & FIBRINOLYSIS, RAPID COMMUNICATIONS, OXFORD,OXFORD, GB, Bd. 10, Nr. 8, Dezember 1999 (1999-12), Seiten 471-479, XP001036685 ISSN: 0957-5235
- PRINS T.W. ET AL: 'Cloning and characterization of a glutathione S-transferase homologue from the plant pathogenic fungus Botrytis cinerea' MOLECULAR PLANT PATHOLOGY Bd. 1, Nr. 3, Mai 2000, Seiten 169 - 178

## Beschreibung

Die Erfindung betrifft Mutanten der den Blutgerinnungsfaktor VII aktivierenden Protease (FSAP), Verfahren zum Nachweis dieser Mutanten auf Protein- sowie RNA/DNA-Ebene oder in einer Gewebeprobe.

Aus der deutschen Patentanmeldung DE 199 03 693 ist bereits eine aus dem Blutplasma isolierte Protease bekannt, die den Gerinnungsfaktor VII aktivieren kann. Aufgrund dieses ersten Befundes wurde sie als Faktor VII aktivierende Protease (FSAP) bezeichnet. Detaillierte Untersuchungen zeigten, dass FSAP auch ein potenter Aktivator von Einketten-Plasminogenaktivatoren ist wie Prourokinase oder Einketten-Gewebe-Plasminogenaktivator (sct-PA). Aufgrund dieser Eigenschaften wurden Anwendungsmöglichkeiten von FSAP beschrieben, beispielsweise ihre Anwendung als gerinnungsfördemdes Mittel basierend auf der durch FVII-Aktivierung unterstützten Beschleunigung der Coagulation. Allein oder in Kombination mit Plasminogenaktivatoren kann FSAP auch zur Fibrinolyse Anwendung finden, beispielsweise bei thrombotischen Komplikationen.

Wie in den deutschen Patentanmeldungen DE 199 03 693 und 199 26 531 beschrieben, wurden Tests zur Detektion der Protease entwickelt, die sowohl die Quantifizierung des FSAP Antigengehaltes sowie deren Aktivität zum Beispiel im Plasma ermöglichen. Die Antigenbestimmung wird dabei vorzugsweise mittels eines ELISA-Tests durchgeführt. Die FSAP-Aktivität kann - wie in der deutschen Patentanmeldung DE 199 26 531 beschrieben - durch Quantifizierung der Aktivierung von Prourokinase zur Urokinase und deren Umsetzung mit einem chromogenen Substrat mit anschließender Differenzmessung der Extinktion bestimmt werden. Ein überraschender Befund bei Durchführung dieses Aktivitätstestes war, dass das aus zum Beispiel Plasma isolierte FSAP Proenzym bei den gewählten Inkubationsbedingungen aktiviert wurde und so die Aktivierung der Prourokinase ermöglichte. Neuere Untersuchungen haben gezeigt, dass FSAP durch Eigenaktivierung in die aktivierte Form überführt wird und so beispielsweise Prourokinase oder den FVII aktivieren kann. Dies wird durch die oben genannten Inkubationsbedingungen, nämlich neutraler bis alkalischer pH-Wert, Kalziumionen und Heparin noch unterstützt. Zudem weisen jüngste Ergebnisse darauf hin, dass Prourokinase/Urokinase (oder Ein- und Zweiketten-tPA) selbst eine Aktivierung von Einketten-FSAP hervorrufen oder unterstützen.

Unter Anwendung der beiden vorstehend genannten Testsysteme, nämlich dem ELISA- und dem Prourokinase-Aktivierungstest, wurden mehr als 180. Plasmen gesunder Blutspender untersucht. Dabei zeigte sich, dass 5 bis 10% aller Proben gegenüber einem Plasmapool (aus mehr als 100 gesunden Spendern) oder dem Durchschnitt des gesamten Testkollektives eine deutlich erniedrigte Potenz der durch FSAP bewirkten Prourokinase-Aktivierung aufweisen.

Dagegen wurden in der Mehrzahl dieser Spender (mit erniedrigter Aktivität) durchschnittliche FSAP-Antigenwerte gemessen. Es wird daher vermutet, dass in den untersuchten Blutproben eine oder mehrere Modifikationen des FSAP enthalten sein könnten, die eingeschränkte oder fehlende Aktivitäten zur Folge hätten. Dies könnte in Polymorphismen in der Bevölkerung, also einer oder mehrerer Mutationen in den FSAP-Strukturen, die sich in einer Änderung der FSAP-Aminosäuresequenz zeigen, begründet sein, wie schon in der deutschen Patentanmeldung DE 199 26 531 vermutet wurde. Die in der Regel um 50 bis 70% gegenüber dem Durchschnittswert aller untersuchten Spender erniedrigten Aktivitäten weisen auf eine heterozygote Mutation hin. Dies könnte sich phenotypisch durch wahrscheinlich paritätische Anwesenheit beider FSAP's, nämlich der Wildtyp-FSAP und der mutanten Variante, im Plasma ausprägen. Angenommen, die mutierte Variante hätte die Eigenschaft (nahezu) völlig eingebüßt, Prourokinase zu aktivieren, so würde im Mittel eine ungefähr halbierte Aktivität messbar werden. Darüber hinaus wurden jedoch auch schon pseudohomozygote Ausprägungen heterozygoter Mutationen anderer Proteine beschrieben, bei denen lediglich das mutierte Protein detektierbar war, welches aber als solches nur einen Teil der entsprechend detektierten biologischen Eigenschaft eingebüßt hatte.

Um auszuschließen, dass der Mangel oder die Verminderung unbekannter potentieller Kofaktoren für die festgestellte Einbuße der FSAP-Aktivität verantwortlich ist, wurden FSAP-Proben von drei Spendern gereinigt, die bei wiederholten Spenden eine signifikant erniedrigte Aktivität gezeigt hatten. Die hochgereinigten Proteine zeigten gegenüber der aus dem Plasmapool gereinigten FSAP ebenfalls eine deutlich verminderte Aktivität. Dies reduziert die Wahrscheinlichkeit eines Kofaktoreinflusses und erhöht die einer Proteinmodifikation im oben genannten Sinne. Überraschend war der Befund, dass die Potenz zur Aktivierung von Faktor VII nicht eingeschränkt zu sein scheint. Aus diesem Grunde sind solche Mutanten besonders für die oben genannte Anwendung als gerinnungsfördemdes Mittel - wie in der deutschen Patentanmeldung DE 199 03 693 beschreiben - geeignet, da deren fibrinolytisches Potential offenbar limitiert ist. Diese Mutanten können basierend auf den im folgenden beschriebenen Erkenntnisse der Nukleotidsequenz-Änderungen rekombinant oder transgen hergestellt werden. Sie können aber auch ebenso wie das entsprechende FSAP-Protein (Ein- oder Zweiketten-FSAP) aus natürlichen Quellen wie Blutplasma direkt isoliert werden. In den deutschen Patentanmeldungen DE 199 03 693, 199 37 219 und 199 37 218 wurden bereits Verfahren beschrieben, die die Herstellung von FSAP erlauben, bevorzugt mit Hilfe der Immunabsorption, wie es in der deutschen Patentanmeldung DE 100 36 641 im einzelnen erläutert ist. Die bisher verwendeten monoklonalen Antikörper unterscheiden jedoch, so weit bekannt, nicht zwischen dem Wildtyp und den Mutanten des FSAP. Entsprechend können nur monoklonale Antikörper, die spezifisch mit den Mutanten reagieren, zur Herstellung der Mutanten verwendet werden. Dabei können die Antikörper durch Immunisierung mit der Mutante gewonnen werden. Außerdem können Peptide mit Proteinregionen, die den Aminosäuren 389 bis 397 ( ... SFRVQKIFK ... ) und/oder 534 bis 539 (...EKRPGV...) der SEQ. ID No. 4 des Sequenzprotokolls entsprechen, nach bekannten Methoden zur Immunisierung und Generierung entsprechender Antikörper verwendet werden. Außerdem finden diese Antikörper auch Anwendung zur spezifischen Detektion dieser Mutanten, zum Beispiel als Reagenzien in Nachweisverfahren wie ELISA, Western Blots, in der lmmunhistologie oder beim Fluorescence Assisted Cell Sorting (=FACS).

Dagegen können Antikörper, die spezifisch für den FSAP-Wildtyp bzw. gegen die entsprechenden Aminosäuresequenz des Wildtyps gerichtet sind, zum Beispiel gegen die Aminosäuresequenzen 389 bis 397 (...SFRVEKIFK...) und/oder gegen die Aminosäuresequenz 534 bis 539. (...GKRPGV...) gerichtet sind vor allem in humanisierter Form als Pharmazeutikum zur prophylaktischen oder therapeutischen Inhibition der FSAP-Aktivität verwendet werden, um beispielsweise Blutungen zugrundeliegenden Hyperfibrinolysen entgegenzuwirken. Außerdem können diese Antikörper auch zur Reinigung, Detektion und Differenzierung der Wildtyp-FSAP in der oben beschriebenen Weise verwendet werden.

Die genomische Sequenz des FSAP wurde in der Genbank unter der Accession No. AC 006097 durch Abgleich mit der bekannten cDNA-Sequenz (Choi-Miura, Accession No. S 83182) identifiziert und dabei Intron- und Exon-Sequenzen abgeleitet. Insgesamt wurden 12 Primerpaare entworfen, um die kodierenden Sequenzen in spezifischen PCR-Reaktionen zusammen mit einem kleinen Teil der jeweils flankierenden Intron-Sequenzen amplizieren zu können.

Zunächst wurde die genomische DNA aus Blut von 2 Probanden mit erniedrigter und von 4 Probanden mit normaler Prourokinase-Aktivität isoliert mit allen Primerpaaren amplifiziert und anschließend unter Verwendung der PCR-Primer die DNA-Sequenz bestimmt. Das Ergebnis ist in Tabelle 1 dargestellt. Insgesamt 4 Nukleotidpositionen in der kodierenden Region waren polymorph, d.h. an diesen Stellen werden zwei Basen gleichzeitig nachgewiesen. Es ist daher davon auszugehen, dass in diesen Fällen Heterozygosität vorliegt, mit einem Wildtyp- und einem mutanten Allel. Zwei davon (an Position 183 und 957) sind Drittbasenaustausche, die nicht zu einem Aminosäureaustausch führen. Die beiden anderen, die nur in der DNA der Probanden mit erniedrigter Proruokinase-Aktivität gefunden wurden, führen zu Aminosäureaustauschen wie in Tabelle 1 dargestellt.

**Tabelle 1**

| **DNA-Sequenz an Nukleotidpositionen*** | | | | | |
|---|---|---|---|---|---|
| Proband Nr. | ProUK-Aktivität | 183 | 957 | 1177 | 1601 |
| S83182 | | T | G | G | G |
| 9689 | normal | T/C | G | G | G |
| 9690 | normal | T/C | G | G | G |
| 9704 | normal | T | G/A | G | G |
| 9706 | normal | T | G/A | G | G |
| 9714 | erniedrigt | T | G | G/C | G/A |
| 9715 | erniedrigt | T | G | G/C | G/A |

| **Aminosäure an Position*** | | | | | |
|---|---|---|---|---|---|
| Proband Nr. | ProUK-Aktivität | NT*: 183 | NT: 957 | NT: 1177 | NT. 1601 |
| | | AS*: 61 | AS: 319 | AS: 393 | AS: 534 |
| S83182 | | His | Lys | Glu | Gly |
| 9689 | normal | His | Lys | Glu | Gly |
| 9690 | normal | His | Lys | Glu | Gly |
| 9704 | normal | His | Lys | Glu | Gly |
| 9706 | normal | His | Lys | Glu | Gly |
| 9714 | erniedrigt | His | Lys | Glu/Gln | Gly/Glu |
| 9715 | erniedrigt | His | Lys | Glu/Gln | Gly/Glu |

| | | | | | |
|---|---|---|---|---|---|
| * wobei 1 das A des Initationskodons ist | | | | | |
| *NT - Nukleotidposition; AS - Aminosäureposition | | | | | |

Um die Korrelation der beiden Mutationen mit erniedrigter Prourokinase-Aktivität zu untersuchen, wurden die DNAs weiterer Personen an diesen Stellen sequenziert. Das Ergebnis ist in Tabelle 2 zusammengefasst. Alle 6 Probanden mit erniedrigter Prourokinase-Aktivität waren heterozygot an der Nukleotidposition 1601 (Gly - Glu Austausch), vier hatten zusätzlich die Heterozygosität an der Position 1177 (Glu - Gln Austausch). Keiner der insgesamt 11 Probanden mit normaler oder am unteren Normalbereich befindlicher Prourokinase-Aktivität wies die oben genannte Heterozygositäten auf. Dieses Ergebnis lässt darauf schließen, dass zumindest der Austausch an Aminosäureposition 534 ursächlich mit der erniedrigten Prourokinase-Aktivität zusammenhängt. Ob ein Aminosäureaustausch allein in der Position 393 eine Emiedrigung der Prourokinase-Aktivität zur Folge haben könnte, ist derzeit noch ungewiss.

**Tabelle 2**

| **DNA-Sequenz an Nukleotidposition** | | | |
|---|---|---|---|
| Proband Nr. | ProUK-Aktivität | 1177 | 1601 |
| 9714 | Niedrig | C/G | A/G |
| 9715 | Niedrig | C/G | A/G |
| 9802 | Niedrig | C/G | A/G |
| 10032 | Niedrig | G | A/G |
| 10039 | Niedrig | C/G | A/G |
| 10047 | Niedrig | G | A/G |
| 9698 | Unterer Normalbereich | G | G |
| 9702 | Unterer Normalbereich | G | G |
| 9711 | Unterer Normalbereich | G | G |
| 9712 | Unterer Normalbereich | G | G |
| 10038 | Unterer Normalbereich | G | G |
| 9689 | Normal | G | G |
| 9690 | Normal | G | G |
| 9704 | Normal | G | G |
| 9706 | Normal | G | G |
| 9803 | Normal | G | G |
| 10043 | Normal | G | G |

Gegenstand der Erfindung ist somit eine Mutante der DNA-Sequenz, die für die den Blutgerinnungsfaktor VII und Einketten-Plasminogenaktivatoren aktivierende Protease (FSAP) kodiert, die an der Nukleotidposition 1177 einen G/C-Basenaustausch und/oder an der Nukleotidposition 1601 einen G/A-Basenaustausch aufweist.

Die Nukleotidsequenz SEQ. ID No. 1 des beiliegenden Sequenzprotokolls gibt die Sequenz des Wildtyps wieder. Die DNA-Sequenz der Mutante mit beiden Austauschen an den Nukleotidpositionen 1177 und 1601 ist durch die SEQ. ID No. 2 des Sequenzprotokolls beschrieben. Die entsprechende Aminosäuresequenz des Wildtyps kann der SEQ. ID No. 3 des Sequenzprotokolls entnommen werden. Die SEQ. ID No. 4 zeigt die Aminosäuresequenz der Mutante mit den beiden Aminosäureaustauschen (Glu - Gln 393 und Gly - Glu 534).

Mit dem Auffinden der im Sequenzprotokoll genannten DNA- und Aminosäuresequenzen sind die Voraussetzungen zur Entwicklung diagnostischer Verfahren zum Erkennen von Patienten mit genetisch bedingter hetero- oder homozygoter Expression des FSAP geschaffen worden. Man kann die Mutationen entweder in der genomischen DNA oder in der daraus abgeleiteten mRNA nachweisen.

Monoklonale Antikörper gegen die den Blutgerinnungsfaktor VII aktivierende Protease und ihre Mutanten sowie ihre Verwendung zum Nachweis und zur Bestimmung der Aktivität von FSAP und ihren Mutanten werden beschrieben.

An sich sind Testsysteme, die eine Quantifizierung sowohl des FSAP Antigengehalts als auch die Bestimmung seiner Aktivität ermöglichen, wie Western Blots oder ein Enzym-Immunoassay (ELISA) bereits aus den deutschen Patentanmeldungen DE 199 03 693 und 199 26 531 bekannt.

Diese Testsysteme basieren darauf, dass FSAP durch spezifische monoklonale Antikörper gebunden und/oder detektiert wird. Obwohl bei der Immunisierung beispielsweise von Mäusen häufig viele positive Klone bezüglich der Expression eines spezifischen monoklonalen Antikörpers identifiziert werden, sind doch nicht selten nur wenige davon für die vorstehend genannten Fragestellungen geeignet. Es stelle sich deshalb die Aufgabe, spezifische monoklonale Antikörper gegen die den Blutgerinnungsfaktor VII aktivierende Protease aufzufinden, die sowohl für ihre Reindarstellung, als auch für ihren qualitativen und quantitativen Nachweis als auch für ihre Aktivitätsbestimmung effektiv einsetzbar sind.

Es wurde nun gefunden, dass diesen Anforderungen in hohem Maße monoklonale Antikörper gegen die den Blutgerinnungsfaktor VII aktivierende Protease gerecht werden, die von der Hybridomazeillinie DSM ACC2453 und der Hybridomazeillinie DSM-ACC2454 gebildet werden. Diese Antikörper wurden gemäß Budapester Vertrag bei der Deutschen Sammlung von Mikroorganismen und Zell Kulturen in Braunschweig hinterlegt.

Bis vor kurzem wurde FSAP überwiegend in ihrer aktivierten Form gereinigt. Konventionelle Präparationsmethoden, wie säulenchromatografische Techniken, begünstigen die rasche Aktivierung und anschließende Inaktivierung der Protease. Nun ist die Reindarstellung der den Blutgerinnungsfaktor VII aktivierenden Protease und vor allem ihres Proenzyms mit hoher Ausbeute dadurch möglich, dass man einen der vorstehend genannten Antikörper an einen Träger koppelt, diesen mit einer die Protease oder ihr Proenzym enthaltenden Flüssigkeit equilibriert, anschließend auswäscht und dann die Protease oder ihr Proenzym durch Elution gewinnt.

Darüber hinaus eignen sich die genannten Antikörper auch zum Nachweis der den Blutgerinnungsfaktor VII aktivierenden Protease oder ihres Proenzyms mit einem Immunoassay, bei dem man
a) eine Probe, die die Protease oder ihr Proenzym enthalten soll, mit einem auf einem festen Träger fixierten ersten Antikörper inkubiert und auswäscht, dann einen zweiten markierten Antikörper zugibt und abermals auswäscht und das vom zweiten Antikörper oder anderen monoklonalen oder polyklonalen Antikörpern hervorgerufene Signal misst; oder
b) auf einem Träger die in der zu untersuchenden Probe enthaltene Protease oder ihr Proenzym fixiert, beispielsweise durch polyklonalen Antikörper gegen die Protease oder ihr Proenzym, und sie mit einem markierten Antikörper allein oder in Mischung mit einem unmarkierten der Antikörper und anschließendem Nachweis des monoklonalen Antikörpers detektiert oder
c) einen auf einem Träger fixierten Antikörper mit einer auf die Protease oder ihr Proenzym zu untersuchenden Probe in Gegenwart einer markierten Protease oder ihres Proenzyms versetzt und das durch die Markierung hervorgerufene Signal misst.

Eine andere Möglichkeit zum Nachweis der den Blutgerinnungsfaktor VII aktivierenden Protease oder ihres Proenzyms besteht darin, dass man den Nachweis mit der Western Blot-Methodik durch eine immunologische Reaktion mit einem markierten Antikörper allein oder in Mischung mit einem unmarkierten Antikörper und anschließendem Nachweis des monoklonalen Antikörpers, zum Beispiel durch markiertes Protein A oder G oder einen markierten, gegen den monoklonalen Antikörper gerichteten monoklonalen oder polyklonalen Antikörper durchführt.

Schließlich ist es auch möglich, die Aktivität der den Blutgerinnungsfaktor VII aktivierenden Protease oder ihres Proenzyms in Proteinlösungen dadurch zu bestimmen, dass man die die Protease und/oder ihr Proenzym enthaltende Proteinlösung an einem festen Träger inkubiert, an die zuvor ein gegen die Protease gerichteter monoklonaler Antikörper gekoppelt wurde und nach Auswaschen des festen Trägers die daran fixierte Protease und ihr Proenzym mit Reagentien inkubiert, die deren Aktivitätsbestimmung erlauben. Die Aktivität der Protease oder ihres Proenzyms kann dann durch eine photometrische Bestimmung der bei der Einwirkung auf chromogene Substrate auftretenden Extinktion gemessen werden.

Andere Möglichkeiten zur Bestimmung der Aktivität der Protease oder ihres Proenzyms bestehen darin, dass man
- ihre die Blutgerinnungsfaktoren VIII/VIIIa oder V/Va inaktivierende Wirkung oder
- ihre die Blutgerinnungszeiten verkürzende Wirkung in globalen Gerinnungstests oder
- ihre Plasminogenaktivatoren aktivierende Wirkung oder
- ihre den Blutgerinnungsfaktor VII aktivierende Wirkung misst.

Für die Anwendung in den vorstehend genannten Verfahren eignen sich sowohl die vollständigen monoklonalen. Antikörper als auch deren Fragmente wie F(ab')₂ oder Fab. Die Antikörper oder ihre Fragmente werden nach Markierung mit einer radioaktiven oder fluoreszierenden oder enzymatisch aktiven Substanz als detektierende Hilfsmittel in einem Immunoassay oder in einem Western Blot-Nachweisverfahren eingesetzt. Die unmarkierten monoklonalen Antikörper oder Fragmente können ebenfalls eingesetzt werden, jedoch wird dann die Detektion oder Immobilisierung zum Beispiel durch einen gegen den Maus-Antikörper gerichteten markierten Antikörper oder sein markiertes Fragment durchgeführt (Sandwich-Verfahren). Die monoklonalen Antikörper oder ihre Fragmente können allein oder als Mischung eingesetzt werden. Dies ist besonders empfehlenswert bei Western Blots, da SDS-Gelelektrophoresen häufig unter reduzierenden Bedingungen der Proben durchgeführt werden. Da die beiden Polypeptidketten der FSAP lediglich über eine Disulfidbrücke miteinander verbunden sind, zerfällt das Molekül bei der Reduktion in zwei Ketten, nämlich in die schwere und die leichte Kette, wobei die erstere vom monoklonalen Antikörper der DSM ACC2454 und die letztere vom monoklonalen Antikörper der DSM ACC2453 erkannt wird. Zur Detektion beider Ketten sind also die beiden monoklonalen Antikörper oder ihre Fragmente erforderlich.

Die monoklonalen Antikörper wurden wie folgt hergestellt und charakterisiert:

### Immunisierung

Drei weibliche Balb/c-Mäuse (ca. 6 Wochen alt) wurden mit FVII-Aktivator immunisiert. Die erste Injektion bestand aus 0,2 ml des Antigens (10 µg) gemischt mit 0,2 ml kompl. Freund's Adjuvans. In den drei folgenden Boost-Injektionen (Abstand jeweils 2 Wochen) wurde das Antigen (20 µg in 0,2 ml) ohne Adjuvans verabreicht (alle Injektionen i.p.). Die Verdünnung des Immunogens erfolgte in PBS. Nach der letzten Injektion wurden die Serum-Titer mittels indirektem ELISA bestimmt, indem eine Mikrotiterplatte mit FVII-Aktivator beschichtet wurde. Für die Fusion wurde die Maus mit dem höchsten Serum-Titer ausgewählt.

### Fusion

Etwa drei Wochen nach der letzten Applikation wurde das Antigen an drei aufeinanderfolgenden Tagen verabreicht (10 µg in 0,1 ml i.v.). Am nächsten Tag (Tag 4) wurde die Maus nach einer Blutentnahme getötet, die Milz entnommen und die Milzzellen isoliert. Die Milzzellen wurden anschließend mit der murinen Myelom-Zelllinie SP2/0-Ag 14 fusioniert. Als Fusionsreagenz wurde Polyethylenglykol 4000 (Merck) verwendet. Die Fusion wurde mit einer Modifikation der Original Köhler/Milstein-Methode durchgeführt. Die Zellen wurden auf 24 Well-Kulturplatten verteilt. Als Medium wurde Dulbecco mod. Eagle's Medium mit 10% fötalem Kälberserum und HAT zur Selektion eingesetzt. Nach etwa zwei Wochen wurden die gewachsenen Zellklone in die Vertiefungen einer 48 Well-Platte transferiert und kodiert.

### Hybridoma-Screening

Von 1728 gewachsenen Klonen wurde der Kulturüberstand genommen und mittels ELISA auf Anwesenheit von Maus IgG getestet. Mit Hilfe von immobilisiertem FVII-Aktivator wurden Maus IgG positive Überstände auf Spezifität geprüft (ELISA). Von den getesteten Zelllinien wurden 108 Zelllinien als spezifisch für FVII-Aktivator identifiziert und kryokonserviert.

Die zwei Hybridoma-Zelllinien mit den Bezeichnungen DSM ACC2453 und DSM ACC2454 wurden für weitere Untersuchungen ausgewählt. Die Spezifität der von diesen Zelllinien gebildeten Antikörper wurde mit dem BIACORE bestätigt und die Bindungskinetik untersucht. Die beiden monoklonalen Antikörper sind vom Typ des IgG1.

Mit Hilfe der beschriebenen Antikörper gegen den FSAP-Wildtyp und gegen ihre Mutanten können diagnostische Verfahren zum Nachweis der Mutanten durchgeführt werden.
So kann
a) eine Probe, die die Mutante enthalten könnte, mit einem auf einem festen Träger fixierten ersten Antikörper inkubiert und danach wäscht, dann einen zweiten, markierten Antikörper oder gegen den Wildtyp gerichteten markierten Antikörper zugibt und abermals auswäscht und das vom zweiten Antikörper hervorgerufenen Signal misst oder
b) eine Probe, die die Mutante enthalten könnte, mit einem auf einem festen Träger fixierten ersten Antikörper gegen den Wildtyp inkubiert und danach wäscht, dann einen zweiten, markierten Antikörper zugibt und abermals auswäscht und das vom zweiten Antikörper hervorgerufenen Signal misst oder
c) auf einen Träger die auf das Vorliegen der Mutante zu untersuchende Probe fixiert und sie mit einem markierten Antikörper allein oder in Mischung mit einem unmarkierten Antikörper und anschließendem Nachweis des markierten Antikörpers detektiert oder
d) einen auf einem Träger fixierten Antikörper mit einer auf das Vorliegen der Mutante zu untersuchenden Probe in Gegenwart einer markierten Mutante versetzt und das durch die Markierung hervorgerufene Signal misst.

Bevorzugt ist ein diagnostisches Verfahren, bei dem man die Aktivität der FSAP misst, indem man die die Protease enthaltende. Probe an einem festen Träger inkubiert, an den zuvor ein gegen die Protease gerichteter Antikörper gekoppelt wurde und nach Auswaschen des festen Trägers die fixierte Protease mit Reagenzien inkubiert, die deren Aktivitätsbestimmung erlauben.

Dabei kann die Aktivität der Protease durch eine photometrische Bestimmung der bei der Einwirkung auf chromogene Substrate auftretenden Extinktion gemessen werden.

Es ist auch möglich, die Aktivität der Protease durch Messung
- ihrer die Blutgerinnungsfaktoren VIII/VIIIa und V/Va inaktivierende Wirkung oder
- ihre die Blutgerinnungszeiten verkürzende Wirkung in globalen Gerinnungstests oder
- ihrer Plasminogenaktivatoren aktivierende Wirkung oder
- ihre den Blutgerinnungsfaktor VII aktivierende Wirkung zu bestimmen.

Schließlich stehen auch Verfahren zur Verfügung, bei denen die die Plasminogenaktivatoren aktivierende Wirkung gemessen wird, durch die Aktivierung der
- Einketten-Urokinase (scuPA, single chain urokinase plasminogen activator) oder des
- Einketten-tPA (sctPA, single chain tissue plasminogen acitivator).

Zum Nachweis der für die Erniedrigung der Prourokinase-Aktivität verantwortlichen Mutationen auf DNA- und RNA-Ebene können Verfahren eingesetzt werden, wie sie auch zum Nachweis von single nucleotid polymorphisms angewendet werden, zum Beispiel
- die cDNA-Amplifikation der RNA oder die Amplifikation der genomischen DNA und ihre anschließende Sequenzierung;
- der Mutationsnachweis auf Ebene der cDNA oder genomischen DNA oder deren Amplifikation durch
- die Hybridisierung mit sequenzspezifischen Sonden, die auch Markierungen zum Nachweis tragen können wie Enzyme, alkalische Phosphatase, HRP und deren Substrate, Fluoreszenzfarbstoffe, auch Reporter-Quencher-Paare (wie zum Beispiel Scorpions, Molecular Beacons, TaqMan-Sonden), radioaktive Atome, Chromophore, Chemo- und Elektrochemolumineszenzmarkierungen) oder
- durch Verfahren wie die selektive 2'-Amin-Acylierung, die elektrochemische Oxidation von Nukleinsäuren, durch "minor groove binder" Oligonukleotid-Konjugate oder durch die HPLC.

Auf der Grundlage der Untersuchungsergebnisse, die durch die vorstehend genannten Antigen- und Aktivitätstests erhalten wurden, konnten drei Gruppen von gesunden Spendern hinsichtlich potentieller Mutationen auf genomischer Ebene untersucht werden. Dazu wurde den Spendern Blut entnommen, und die Blutzellen durch Zentrifugation vom Plasma getrennt. Die Plasmen wurden dann zur Quantifizierung der FSAP-Antigen- und Aktivitätsspiegel verwendet und entsprechend den letzteren in drei Gruppen unterteilt, nämlich in "hoch/durchschnittlich", "durchschnittlich/erniedrigt" und "signifikant erniedrigt". Die gewonnenen Blutzellen wurden dann zur DNA/RNA-Extraktion verwendet und daraus die auf Seite 5 und in Tabelle 1 dargestellten Befunde ermittelt.

Basierend auf den vorliegenden Ergebnissen ist nunmehr die rasche Detektion einer oder beider beschriebenen Mutationen, gleichgültig ob hetero- oder homozygoten Genotyps, auf der Ebene der entsprechenden FSAP-Nukleotidsequenz möglich, Während die vorstehend genannten Antigen- und Aktivitätstests in gesundem Zustand eines Spenders durchaus den Genotyp widerspiegeln, kann dies bei Einflüssen auf die FSAP-Plasmaspiegel schwierig oder unmöglich werden. So können Parameter wie hormonelle Schwankungen, Lebensstil usw. besonders aber Krankheitszustände Antigen- und/oder Aktivitätsspiegel mehr oder minder stark beeinflussen. Wie in der deutschen Patentanmeldung DE 199 26 531 beschrieben, kann bei einem Herzinfarkt die messbare FSAP-Aktivität bei kaum erhöhtem Antigengehalt deutlich gegenüber dem Normalwert ansteigen, wodurch Spender, die im gesunden Zustand eine erniedrigte FSAP-Aktivität aufweisen, nun als "durchschnittlich" erscheinen.

Beispielsweise sind Untersuchungen, ob Patienten mit FSAP-Mutation ein erhöhtes Risiko haben, thrombotische Komplikationen wie Herzinfarkte zu erleiden, aufgrund der vorstehend genannten Beschränkungen nur schwer möglich. Dagegen können beispielsweise Leberinsuffizienzen zu erniedrigten Plasmaspiegeln führen, was ebenfalls zu Missinterpretationen der "wahren" genetischen Prädispositon führen kann. Ein Test auf FSAP-Mutation auf DNA/RNA-Ebene ist dagegen von temporären Ereignissen unabhängig. Die Kombination aller genannten Assays ernnöglicht ein komplettes Bild des Spenders/Patienten, nämlich die Beurteilung einer potentiellen Mutation und des akuten Zustandes hinsichtlich einer Beeinflussung des Antigen-Aktivitätsverhältnisses. Daraus können prophylaktische und therapeutische Maßnahmen resultieren.

Wie vorstehend beschrieben, wurden bisher ausschließlich heterozygote Blutspender gefunden, deren Blutplasma zu etwa 50% die normale FSAP und zu etwa 50% die FSAP-Mutante enthält. Hieraus ergibt sich ein um etwa 50% erniedrigter Aktivitätsspiegel von Plasmen, in denen beide Arten von FSAP-Molekülen vorkommen. Plasmapools, die aus dem Blut von 100 und mehr Spendern gewonnen worden sind, enthalten demnach auch je nach Population 5 bis 10% der FSAP-Mutanten. Hieraus ergibt sich bei Bluttransfusionen eine entsprechende Wahrscheinlichkeit, Blutplasma eines Spenders zu bekommen, welches die FSAP-Mutante enthält. Wird ein diese Mutante enthaltendes Blut einem Empfänger verabreicht, der diese Mutante nicht bildet, dann können diese von ihm als fremd erkannt und entsprechende Antikörper ausgebildet werden. Bei einer späteren Folgeapplikation mit der FSAP-Mutante kann es zu immunologischen Reaktionen des Empfängers mit den dem Fachmann vertrauten Nebenwirkungen kommen.

Umgekehrt wird bei einem homozygoten Blutempfänger, der lediglich die FSAP-Mutante, nicht aber die normale FSAP bildet, diese als "fremd" erkannt und entsprechende Antikörper dagegen gebildet.

FSAP beeinflusst die Hämostase und die damit verbundenen zellulären Prozesse. Durch Involvierung in die Blutgerinnung und/oder die Fibrinolyse beeinflusst es auch die Wundheilungsreaktion. Außerdem kann die FSAP durch ihre Eingenschaft, hohe Affinität zu Glykosaminoglykanen zu besitzen, an Zellen und anderen Matrices gebunden werden, wodurch sie physiologisch und pathophysiologisch möglicherweise an der Zellmigration und zellulär-proteolytischen Prozessen beteiligt ist.

Antikörper gegen FSAP können somit alle durch die FSAP vermittelten Aktivitäten beeinflussen. Für den Fall, dass Autoantikörper gegen die FSAP auftreten, kann neben einer Beeinträchtigung der physiologischen Funktionen noch hinzukommen, dass Immunkomplexe (FSAP + Antikörper) zu Nebenwirkungen bekannter Autoimmunerkrankungen betragen. Das kann beispielsweise lokalisiert am Endothel zu Vaskulitiden führen. Die Neutralisierung der FSAP-Aktivität als profibrinolytisches Agens könnte außerdem zu einem Thrombose-fördernden Zustand beitragen.

Es besteht deshalb der Bedarf an einem diagnostischen Verfahren zum Nachweis der vorstehend beschriebenen Antikörper.

Möglich ist ein diagnostisches Verfahren zum Nachweis von Antikörpern gegen die den Faktor VII aktivierende Protease (=FSAP) und/oder gegen eine durch den Austausch einer oder mehrerer Aminosäuren gebildeten Mutante der FSAP, bei dem man eine Probe, die Antikörper enthaltenkönnte, auf die an einen festen. Träger fixierte FSAP und/oder FSAP-Mutante einwirken lässt, danach wäscht, den an die Protease(n) gebundenen Antikörper mit einem markierten humanen Anti-Immunoglobulin oder einem markierten Protein A inkubiert und das von der gebundenen, markierten Substanz ausgesendete Signal bestimmt.

Dieses diagnostische Verfahren wird zweckmäßig mit der ELISA-Technik durchgeführt. Dabei wird FSAP und/oder die FSAP-Mutante an eine Matrix gebunden, zum Beispiel an eine Mikrotiterplatte. Zur optimalen Präsentation des FSAP und/oder der FSAP-Mutante kann zuvor eine Beschichtung der Platte mit monoklonalen oder polyklonalen Antikörpern oder deren F(ab')₂ oder F(ab') Fragmenten vorgenommen werden, um danach mit FSAP und/oder der FSAP-Mutante beladen zu werden. Da FSAP und die Mutante sehr gut an Dextransulfat, Heparin und ähnliche Substanzen binden, ist auch die vorherige Beschichtungen mit diesen Agenzien zu FSAP-Bindung möglich. Nach Waschen des Trägers oder der Mikrotiterplatte wird diese ggf. noch mit den hierfür bekannten Agenzien wie Detergenz oder Albumin, blockiert, gewaschen und anschließend mit der zu testenden Lösung inkubiert. Bei den FSAP-Antikörpem enthaltenden Lösungen kann es sich um Blutserum, Plasma und andere Körperflüssigkeiten wie die Synovialflüssigkeiten, Liquor, Speichel, Tränen oder Seminalplasma oder auch Zelllysate handeln.

Nach Inkubation und Waschen des Trägers findet dann ein geeignetes Nachweisagenz Verwendung. Die zur Detektion der verschiedenen Antikörperklassen wie IgG, IgM, IgA, IgE und den dazugehörigen Subklassen erforderlichen Testsubstanzen sind als markierte Reagenzien im Handel erhältlich. Der Nachweis und die. Quantifizierung des Antikörpertiter kann durch eine photometrische Bestimmung erfolgen, bei der die Extinktion gemessen wird, die durch die Spaltung eines chromogenen Substrates durch ein an den anti-Human-Antikörper gekoppeltes Enzym bewirkt wird. Es ist aber auch möglich, die Fluoreszenz zu messen, die eine mit dem zum Nachweis verwendeten Antikörper verbundene fluoreszierende Gruppe ausstrahlt. Schließlich ist es auch möglich, den Nachweis mit einer radiometrischen Messung durchzuführen, wenn die zur Detektion verwendete Substanz mit einer radioaktiven Gruppe markiert ist.

Durch die Ermittlung von Antikörpern gegen FSAP und/oder insbesondere FSAP-Mutanten kann vor Durchführung einer Bluttransfusion das damit verbundene Risiko erkannt und durch geeignete Maßnahmen gefährliche Komplikationen vermieden werden.

Antikörper gegen FSAP erlauben ein diagnostisches Verfahren zum immunhistochemischen Nachweis der den Blutgerinnungsfaktor VII aktivierenden Protease, ihres Proenzyms oder ihrer Mutanten oder Spaltstücke, bei dem man auf eine Gewebeprobe einen gegen die Protease gerichteten markierten, monoklonalen oder polyklonalen Antikörper oder eines seiner Fragmente einwirken lässt, den nicht gebundenen Antikörper oder seine Fragmente auswäscht und das von dem gebundenen Antikörper oder einem seiner Fragmente ausgesendete Signal bestimmt.

Das Verfahren kann auch so durchgeführt werden, dass man auf die Gewebeprobe einen gegen die Protease; ihr Proenzym oder seine Mutanten oder Spaltstücke gerichteten unmarkierten monoklonalen oder polyklonalen Antikörper oder eines seiner Fragmente einwirken lässt, den nicht gebundenen Antikörper oder seine Fragmente auswäscht, dann einen markierten anti-Antikörper auf das Gewebe einwirken lässt und nach Auswaschen des nicht gebundenen, markierten anti-Antikörpers das von dem gebundenen anti-Antikörper oder seinen Fragmenten ausgesendete Signal bestimmt.

Es wurde außerdem gefunden, dass gegen FSAP gerichtete monoklonale oder polyklonale Antikörper, wenn sie mit chromophoren oder lumineszierenden Gruppen markiert sind, hervorragend zum Nachweis von FSAP in Gewebeschnitten humanen Ursprungs geeignet sind. Für diesen Nachweis eignen sich polyklonale Antikörper gegen FSAP, die durch Immunisierung von Kaninchen, Schafen, Ziegen oder anderen Säugetieren gewonnen werden, ebenso wie monoklonale Antikörper. Besonders gut geeignet zum histologischen, spezifischen Nachweis von FSAP, die sowohl in der aktivierten Form, als auch in der Proenzymform oder als Spaltstück vorliegen kann, sind die monoklonalen Antikörper der Hybridomazelllinien DSM ACC 2453 und DSM ACC 2454. Auch Komplexe von aktivierter FSAP mit Inhibitoren, wie Antiplasmin, können so detektiert werden Hierfür geeignet sind alle gängigen histologischen Nachweisverfahren wie die Licht-, Fluoreszenz- und Elektronenmikroskopie.

Zum Nachweis von FSAP in den vorstehend genannten Verfahren eignen sich sowohl die vollständigen polyklonalen und monoklonalen Antikörper als auch deren Fragmente wie F(ab')₂ oder Fab, sofern sie mit einer detektierbaren Gruppe markiert sind. Dabei können die vorstehend genannten Antikörper oder ihre Fragmente allein oder als Mischung angewendet werden. Das ist besonders dann empfehlenswert, falls eines der erkannten Epitope verdeckt ist. Beispielsweise kann eine Proteindomäne durch Zellassoziation für einen Antikörper nicht zugänglich sein, wird jedoch durch einen anderen Antikörper mit der Spezifität für eine andere FSAP-Region gebunden. Auch Antikörper, die gegen die humane FSAP, deren Wildtyp und/oder gegen deren Mutanten gerichtet sind und die in der deutschen Patentanmeldung DE 100 52 319 näher beschrieben sind, können für den Nachweis von FSAP in Gewebeschnitten humanen Ursprungs eingesetzt werden.

Die bisher gewonnenen Erkenntnisse über den immunhistochemischen Nachweis von FSAP lassen sich wie folgt zusammenfassen:
- FSAP wird in fast allen der bisher untersuchten humanen Gewebe nachgewiesen;
- Endokrin-aktive Zellen wie die Leydig'schen Zwischenzellen oder die endokrinaktiven Zellen der Langerhans'schen Inseln der Pankreas lassen sich mit Antikörpern, die chromophore Gruppen tragen, sehr stark intrazytoplasmatisch anfärben;
- Epithelien und Endothelien zeigen entsprechend ihrer Lokalisation eine mehr oder weniger starke intra-zytoplasmatische immunreaktion mit Antikörpern gegen FSAP;
- Ganglienzellen und Dendriten der Großhimrinde zeigen eine hohe Konzentration von FSAP, was durch eine starke immunhistologische Farbreaktion mit chromophoren Antikörpern nachgewiesen wird;
- Plasmazeilen zeigen eine intensive intra-zytoplasmatische Verfärbung mit chromophoren Antikörpern;
- mesenchymale Stromazeilen zeigen in komplexen Geweben keine oder nur eine schwache Farbreaktion für FSAP.

FSAP ist somit ein Protein, das als ein normaler Zellbestandteil anzusehen ist. Bislang wurde FSAP sowohl intra- als auch extrazellulär lokalisiert gefunden, wobei das erstere Kompartiment deutlich stärker anfärbbar ist. Durch die Detektion von FSAP mit den genannten Antikörpern oder ihren Fragmenten können folgende pathologische Vorgänge erkannt werden:
- Endokrin-aktive Tumore und neuro-endokrine Tumore;
- angiogene Endothelien und Endothelien des Kapillarendothels; sowie
- angiogenaktive Tumore wie Gliome und Glioblastome, aber auch beispielsweise Gefäßtumore wie das Hämangioendothelium oder -perizyotom und Angiosarkom;
- Wundheilungsreaktionen, Granulationsgewebe und Kollagenosen;
- arteriosklerotische, (mikro)thrombosierte und nekrotisierende Areale;
- neurodegenerative Erkrankungen, wie Alzheimer'sche Krankheit, Morbus Parkinson aber auch spongiforme Enzephalitiden, beispielsweise ausgelöst durch Prionenproteine;
- Gammopathien und Myelome.

Für den Nachweis der FSAP verwendet man vorzugsweise monoklonale Antikörper der Hybridomazeillinien DSM ACC 2453 oder DSM ACC 2454.

Dieses diagnostische Verfahren wird durch das nachfolgende Beispiel näher erläutert:

Zur Untersuchung der immunhistochemischen Reaktivität der für FSAP spezifischen monoklonalen Antikörper der Hybridomazeillinien DSM ACC 2453 und DSM ACC 2454 wurden aus adultem humanem Gewebe und malignen urologischen Tumoren 10 µm dicke Paraffinschnitte mit anschließender Entparaffinierung hergestellt, die im Zitratpuffer für 3 mal 5 Minuten in der Mikrowelle behandelt wurden. Auf diese Schnitte ließ man zunächst für 30 Minuten einen unmarkierten Antikörper der oben genannten Hybridomazeillinien einwirken. Nach Auswaschen des Gewebeschnittes ließ man auf das Gewebe einen markierten Anti-Maus-Detektionsantikörper für ebenfalls 30 Minuten einwirken und machte dann den gebundenen FSAP-Antikörper durch Bildung des APAAP-Komplexes (Alkalische Phosphatase-Anti-Alkalische-Phosphatase-Komplex) und durch Färbung mit Chromogen und Gegenfärbung mit Hämalaun sichtbar.

Als negative Kontrolle wurde in jedem Gewebe separat eine Inkubation mit dem Detektionsantikörper - ohne vorherige Inkubation mit dem FSAP-Antikörper - durchgeführt, um potentielle unspezifische Reaktionen des Detektors sichtbar zu machen. Außerdem wurde als Positivkontrolle ein Antikörper gegen α-Keratin mitgeführt.

Die Ergebnisse der immunhistochemischen Untersuchung normalen Humangewebes sind in Tabelle 1 zusammengefasst:

**Antikörper gegen FSAP, Klon DSMZ ACC2454 und DSMZ ACC 2453 Humanes Normalgewebe**

| | **DSMZ ACC2454** | **DSMZ ACC2453** | | **2454** | **2453** |
|---|---|---|---|---|---|
| **Oesophagus** | | | **Appendix** | | |
| Plattenepithel | 2+ | 2+ | Epithelien | 1+ | 3+ |
| Drüsenendstücke | 0 | 0 | Muskulatur | 1+ | 1+ |
| Schaltstücke | 2+ | 2+ | Lymphfollikel | 1+ | 2+ |
| Muskulatur | 1+ | 1+ | Plasmazellen | 2+ | 3+ |
| Stroma | 1+ | 1+-2+ | Gefäßendothel | 1+ | 1+ |
| Gefäßendothel | 2+ | 2+ | **Pankreas** | | |
| **Kardia (Magen)** | | | Epithelien | 1+ | 2+ |
| Foveolarepithel | 0 | 0 | Langerhans-Inseln | 3+ | 1+ |
| Glandulae cardiacae | 1+ | 2+ | Gangepithel | 2+ | 2+ |
| Mucinöse Drüsenendstücke | 0 | 0 | Gefäßendothel | 1+ | 1+ |
| Belegzellhaltige Drüsen | 3+ | 3+ | **Speicheldrüse** | | |
| Muskulatur | 1+ | 1+ | Muköse Endstücke | 0 | 0 |
| Gefäßendothel | 1 + | 1+ | Seröse Endstücke | 1 + | 1+-2+ |
| **Korpus (Magen)** | | | Schaltstücke | 1+ | 1+ |
| Foveolarepithel | 0 | 0―1+ | Streifenstücke | 1 + | 1 + |
| Korpus- | 2+ | 2+ | Gefäßendothel | 0-1+ | 0-1+ |
| Drüsenkörper | | | | | |
| Muskulatur | 0 | 1+ | **Leber** | | |
| Gefäßendothel | 1+ | 1+ | Hepatozyten | 2+ | 2+ |
| **Duodenum** | | | Gallengänge | 0 | 0 |
| Epitehlien | 0―1+ | 1+ | Gefäßendothel | 1+ | (1+) |
| Brunnersche Drüsen | 0 | 0 | **Gallenblase** | | |
| Muskulatur | O | O―1+ | Epithel | 1+ | 1+ |
| Lymphfollikel | 1+ | 2+ | Muskulatur | 2+ | 1+ |
| Ganglienzellen | 2+ | 3+ | Gefäßendothel | 2+ | 1 + |
| Gefäßendothel | 1 + | 1+ | **Ductus cysticus** | | |
| **Dünndarm** | | | Epithel | 3+ | 3+ |
| Epithelien | 2+ | 3+ | Muskulatur | 2+ | 1+ |
| Muskulatur | 1+ | 1+ | Ganglienzellen | 3+ | 3+ |
| Stroma | 1+ | 2+ | Gefäßendothel | 2+ | 2+ |
| Ganglienzellen | 3+ | 3+ | **Hoden** | | |
| Gefäßendothel | 1+ | 1+ | Leydigsche Zwischenzellen | 3+ | 1+ |
| **Kolon/Rektum** | | | Sertoli-Zellen | 1+-2+ | 1+ |
| Epithelien | 1+ | 1+ | Keimzellen | 1+-2+ | 1+ |
| Lymphfollikel | 1+ | 1+ | Gefäßendothel | 1+ | 1+ |
| Plasmazeilen | 1+ | 1+ | **Rete testis** | | |
| Gefäßendothel | 1+ | 0 | Epithel | 2+ | 2+ |

| | **2454** | **2453** | | **2454** | **2453** |
|---|---|---|---|---|---|
| **Nebenhoden** | | | **Plazenta** | | |
| Ductus epididymidis | 2+ | 2+ | Chorionepithel | 3+ | 2+ |
| Ductulus efferens | 2+ | 2+ | Amnionepithel | 2+ | 2+ |
| Stroma | 1+ | 1+ | Deziduazellen | 2+-3+ | 2+ |
| Gefäßendothel | 1+ | 1+ | Stromazellen | 0 | +/- |
| **Samenblase** | | | Gefäßendothel | 1+ | 1+ |
| Epithel | 2+ | 3+ | **Eihäute** | | |
| Muskulatur | 1+ | 1+ | Amnionepithel | 3+ | 2+ |
| Gefäßendothel | 2+ | 2+ | Deziduazellen | 3+ | 1+ |
| **Ductus deferens** | | | Fibroblasten | 3+ | 1+-2+ |
| Epithel | 2+ | 3+ | **Cervix Uteri** | | |
| Längsmuskelschic ht | 0 | +/- | Drüsenepithel | 0 | 0 |
| Ringmuskelschicht | 2+ | 3+ | Gefäßendothel | 1+ | 0-1 |
| Gefäßendothel | 2+ | 3+ | Stroma | 1+ | 0-1 |
| **Prostata** | | | **Tuba uterina** | | |
| Drüsenepithel | 2+ | 2+ | Epithel | 2+ | 3+ |

| | | | | | |
|---|---|---|---|---|---|
| Muskulatur | 1+ | 1+ | Muskulatur | 0 | 1+ |
| Gefäßendothel | 1+-2+ | 1+-2+ | Gefäßendothel | 1+ | 2+ |
| **Niere** | | | **Mamma** | | |
| Tubuli | 2+ | 1+ | Epithelien Brustdrüsenlobuli | 2+ | 2+ |
| Glomerula | 0 | 0 | Gangepithel Ausführungsgänqe | 2+ | 2+ |
| Markepithel | 1+ | 1+ | Fibroblasten | 0 | 1+ |
| Gefäßendothel | 0-1+ | 0-1+ | Plasmazellen | 2+ | 2+ |
| **Harnblase** | | | Gefäßendothel | 1 + | 0 |
| Urothel | 2+ | 1+-2+ | **Schilddrüse** | | |
| Muskulatur | 2+ | 1+ | Follikelepithel | 2+ | 1+-2+ |
| Plasmazellen | 2+ | 2+ | Stroma | 1+ | 1+ |
| Fibroblasten | 1+-2+ | 1+-2+ | Gefäßendothel | 1+ | 0 |
| Peripherer Nerv | | 0 | **Thymus** | | |
| **Nebenniere** | | | Hassal-Körperchen | 2+-3+ | 2+ |
| Zona glomerulosa | 2+ | 1+ | Follikel | 1+ | 2+ |
| Zona fasciculata | 1+-2+ | (1+) | Mantelzone | (1+) | (1+) |
| Zona reticularis | 3+ | (1+) | Stemhimmelmakrophagen | 1+ | 1+ |
| Mark | 0 | 0 | **Milz** | + | + |
| Gefäßendothel | 1+ | (1+) | **Tonsille** | +/- | +/- |
| **Endometrium** | | | **Lymphknoten** | +/- | +/- |
| Drüsenepithel | 3+ | 2+ | **Kieferhöhle** | | |
| Stromazellen | 0 | 1+ | Respiratorisches Epithel | 2+ | 2+ |
| Myometrium | 1+ | 1+ | Plasmazellen | 3+ | 3+ |
| Gefäßendothel | 1+ | 2+ | Gefäßendothel | 1+ | 1+ |

| | **2454** | **2453** | | **2454** | **2453** |
|---|---|---|---|---|---|
| **Lunge** | | | **Fettgewebe** | 2+ | 2+ |
| Bronchialepithel | 2+ | 1+ | Gefäßendothel | 2+ | 2+ |
| Alveolarepithel | 1+-2+ | 1+ | **Haut** | | |
| Bronchialdrüsen | 1+ | 1+ | Epidermis | 2+ | 1+-2+ |
| Knorpel | 3+ | 1+ | Dermis | (1+) | 0 |
| Muskulatur | 1+ | 1+ | Subkutis | (1+) | 0 |
| Alveolarmakrophagen | 2+ | 2+ | Schweißdrüsen | 1 + | 0 |
| Elastische Fasern | 2+-3+ | 2+-3+ | Gefäßendothel | 1+ | 0 |
| Gefäßendothel | 1+ | 1+ | **Endokard** | 0 | 0 |
| **Skelettmuskulatur** | 2+ | 1+ | Fibroblasten | 2+-3+ | 2+-3+ |

| | | | | | |
|---|---|---|---|---|---|
| 0 = negativ | | | | | |
| 1+ = schwach positiv | | | | | |
| 2+ = mäßig stark positiv | | | | | |
| 3+ = stark positiv | | | | | |

Bei endokrinen Zellen wie den Langerhans'schen Inseln des Pankreas, den Leidig'schen Zwischenzeilen des Hodeninterstitiums, bei den Deziduazellen der Plazenta und dem beleghaltigen Drüsenkörper der Magenkardia sowie dem hochzylindrischen Epithel des Ductus cysticus zeigt sich eine starke, teils feingranuläre Reaktion. Stark positive Reaktionen wurden in Gewebeverbänden gelegenen Plasmazeilen und gangligen Zellen und den Nervenzellen der Großhimrinde beobachtet. Auch die dezidualen Zellen, das Amnioepithel, und die Fibroplasten der Eihäute zeigten eine sehr starke immunhistologische Anfärbbarkeit, wie auch das auskleidende Epithel der Samenbläschen und die Enterozyten des Dünndarms.

Untersuchungen Formalin-fixierten, in Paraffin eingebetten Tumormaterials urologischer Tumoren zeigten eine schwache bis mäßig starke intra-zytoplasmatische Reaktion unterschiedlich differenzierter Adenokarzinome der Prostata. Tumorzellen seminomatöser Hodentumore zeigten nur eine schwache intra-zytoplasmatische Reaktion, während nicht-seminomatöse Tumore (Embryokarzinome und Chorionkarzinome) eine deutlich verstärkte Anfärbbarkeit der Tumorzellen aufwiesen, die auf erhöhte Konzentrationen von FSAP hinwiesen.

Dieses diagnostische Verfahren erlaubt somit einen immunhistochemischen Nachweis von pathologischen Vorgängen in den verschiedensten Organen.

### SEQUENZPROTOKOLL

<110> Aventis Behring GmbH
<120> Mutanten der den Faktor VII aktivierenden Protease, Verfahren zu deren Detektion und Verwendung
<130> 2000/A008-A7
< 140>
   <141>
<160> 4
<170> Patentin Ver. 2.1
<210> 1
   <211> 1683
   <212> DNA
   <213> Homo sapiens
<400> 1
   atgtttgcca ggatgtctga tctccatgtt ctgctgttaa tggctctggt gggaaagaca 60
   gcctgtgggt tctccctgat gtctttattg gaaagcctgg acccagactg gacccctgac 120
   cagtatgatt acagctacga ggattataat caggaagaga acaccagtag cacacttacc 180
   catgctgaga atcctgactg gtactacact gaggaccaag ctgatccatg ccagcccaac 240
   ccctgtgaac acggtgggga ctgcctcgtc catgggagca ccttcacatg cagctgcctg 300
   gctcctttct ctgggaataa gtgtcagaaa gtgcaaaata cgtgcaagga caacccatgt 360
   ggccggggcc aatgtctcat tacccagagt cctccctact accgctgtgt ctgtaaacac 420
   ccttacacag gtcccagctg ctcccaagtg gttcctgtat gcaggccaaa cccctgccag 480
   aatggggcta cctgctcccg gcataagcgg agatccaagt tcacctgtgc ctgtcccgac 540
   cagttcaagg ggaaattctg tgaaataggt tctgatgact gctatgttgg cgatggctac 600
   tcttaccgag ggaaaatgaa taggacagtc aaccagcatg cgtgccttta ctggaactcc 660
   cacctcctct tgcaggagaa ttacaacatg tttatggagg atgctgaaac ccatgggatt 720
   ggggaacaca atttctgcag aaacccagat gcggacgaaa agccctggtg ctttattaaa 780
   gttaccaatg acaaggtgaa atgggaatac tgtgatgtct cagcctgctc agcccaggac 840
   gttgcctacc cagaggaaag ccccactgag ccatcaacca agcttccggg gtttgactcc 900
   tgtggaaaga ctgagatagc agagaggaag atcaagagaa tctatggagg ctttaagagc 960
   acggcgggca agcacccatg gcaggcgtcc ctccagtcct cgctgcctct gaccatctcc 1020
   atgccccagg gccacttctg tggtggggcg ctgatccacc cctgctgggt gctcactgct 1080
   gcccactgca ccgacataaa aaccagacat ctaaaggtgg tgctagggga ccaggacctg 1140
   aagaaagaag aatttcatga gcagagcttt agggtggaga agatattcaa gtacagccac 1200
   tacaatgaaa gagatgagat tccccacaat gatattgcat tgctcaagtt aaagccagtg 1260
   gatggtcact gtgctctaga atccaaatac gtgaagactg tgtgcttgcc tgatgggtcc 1320
   tttccctctg ggagtgagtg ccacatctct ggctggggtg ttacagaaac aggaaaaggg 1380
   tcccgccagc tcctggatgc caaagtcaag ctgattgcca acactttgtg caactcccgc 1440
   caactctatg accacatgat tgatgacagt atgatctgtg caggaaatct tcagaaacct 1500
   gggcaagaca cctgccaggg tgactctgga ggccccctga cctgtgagaa ggacggcacc 1560
   tactacgtct atgggatagt gagctggggc ctggagtgtg ggaagaggcc aggggtctac 1620
   acccaagtta ccaaattcct gaattggatc aaagccacca tcaaaagtga aagtggcttc 1680
   taa 1683
<210> 2
   <211> 1683
<212> DNA
   <213> Homo sapiens
<400> 2
<210> 3
   <211 > 560
   <212> PRT
   <213> Homo sapiens
<400> 3
<210> 4
   <211> 560
   <212> PRT
   <213> Homo sapiens
<400> 4

## Patentansprüche

1. Mutante der DNA-Sequenz, die für die den Blutgerinnungsfaktor VII und die Einketten-Plasminogenaktivatoren aktivierende Protease (FSAP) kodiert, **dadurch gekennzeichnet, dass** die Mutante an der Nukleotidposition 1601 einen G/A-Basenaustausch gegenüber der Nukleotidsequenz nach SEQ ID No. 1 aufweist.

2. Mutante nach Anspruch 1, **dadurch gekennzeichnet, dass** sie zusätzlich an der Nukleotidposition 1177 einen G/C-Basenaustausch aufweist.

3. Mutante der FSAP, **dadurch gekennzeichnet, dass** die Mutante an der Aminosäureposition 534 einen Gly/Glu-Austausch gegenüber der Aminosäurensequenz nach SEQ ID No. 3 aufweist.

4. Mutante nach Anspruch 4, **dadurch gekennzeichnet, dass** die Mutante zusätzlich an der Aminosäureposition 393 einen Glu/Gln-Austausch aufweist.

5. Proteine oder Peptide aus der Mutante nach Anspruch 3 bis 4, **dadurch gekennzeichnet, dass** sie die Aminosäuren 534 bis 539 der SEQ ID No.4, enthalten.

6. Diagnostisches in vitro Verfahren zum Erkennen von Personen mit genetisch bedingter hetero- oder homozygoter Expression der Mutante der FSAP gemäß den Ansprüchen 1 bis 4, **dadurch gekennzeichnet, dass** man die Mutante der genomischen DNA oder der davon abgeleiteten mRNA nachweist.

7. Diagnostisches in vitro Verfahren zum Erkennen von Personen mit genetisch bedingter hetero- oder homozygoter Expression der Mutante der FSAP gemäß den Ansprüchen 1 bis 4, **dadurch gekennzeichnet, dass** man die Mutante der genomischen DNA nachweist, wobei die genomische DNA aus Blut isoliert wurde.

8. Diagnostisches in vitro Verfahren zum Erkennen von Personen mit genetisch bedingter hetero- oder homozygoter Expression der Mutante der FSAP gemäß den Ansprüchen 1 bis 4, **dadurch gekennzeichnet, dass** man die Aktivität der FSAP Mutante misst, indem man die die Protease enthaltende Probe an einem festen Träger inkubiert, an den zuvor ein gegen die Protease gerichteter Antikörper, sein Fab oder sein F(ab')₂-Fragment, gekoppelt wurde, und nach Auswaschen des festen Trägers die daran fixierte Protease mit Reagenzien inkubiert, die deren Aktivitätsbestimmung erlauben.

9. Diagnostisches Verfahren gemäß Anspruch 8, **dadurch gekennzeichnet, dass** der gegen die den Blutgerinnungsfaktor VII aktivierende Protease (FSAP) gerichtete Antikörper **dadurch gekennzeichnet ist, dass** er von einer der Hybridomazeillinien DSM ACC 2453 oder 2454 gebildet wird.

10. Verwendung der Mutanten nach den Ansprüchen 1 bis 4 zur Herstellung eines Medikaments zur Prophylaxe und/oder Therapie von Blutungen bei angeborenem oder erworbenem Mangel von FVIII, von Willebrand Faktor FV, FIX, FX, FXI, FXII und/oder gegen diese Proteine gerichteten Antikörpern.

11. Verfahren zur Herstellung monoklonaler Antikörper gegen die FSAP Mutante gemäß einem der Ansprüche 3 und 4, **dadurch gekennzeichnet, dass** in einem an sich bekannten Verfahren zur Immunisierung Peptide gemäß Anspruch 5 verwendet werden.

12. Sequenzspezifische Hybridisierungssonde zur Verwendung in einem an sich bekannten Verfahren zum Nachweis eines "single nucleotide polymorphism" in einer FSAP Mutante gemäß einem der Ansprüche 1 bis 2, **dadurch gekennzeichnet, dass** der "single nucleotide polymorphism" ein G/A-Basenaustausch an der Nukleotidposition 1601 ist, wie in SEQ ID No. 2 enthalten.

## Claims

1. A mutant of the DNA sequence coding for the protease (FSAP) which activates blood clotting factor VII and single-chain plasminogen activators, which comprises a G/A base exchange at nucleotide position 1601, compared to the nucleotide sequence of SEQ ID No. 1.

2. The mutant as claimed in claim 1, additionally comprising a G/C base exchange at nucleotide position 1177.

3. An FSAP mutant which comprises a Gly/Glu exchange at amino acid position 534, compared to the amino acid sequence of SEQ ID No. 3.

4. The mutant as claimed in claim 3, which additionally comprises a Glu/Gln exchange at amino acid position 393.

5. A protein or peptide from the mutant as claimed in claims 3 and 4, comprising the amino acids 534 to 539 of SEQ ID No. 4.

6. A diagnostic in vitro method for identifying persons with genetic heterozygous or homozygous expression of the FSAP mutant as claimed in claims 1 to 4, wherein the mutant of the genomic DNA or the mRNA derived therefrom is detected.

7. A diagnostic in vitro method for identifying persons with genetic heterozygous or homozygous expression of the FSAP mutant as claimed in claims 1 to 4, wherein the mutant of the genomic DNA is detected, having been isolated from blood.

8. A diagnostic in vitro method for identifying persons with genetic heterozygous or homozygous expression of the FSAP mutant as claimed in claims 1 to 4, wherein the activity of said FSAP mutant is measured by incubating the sample containing the protease on a solid support to which an antibody directed against said protease, its Fab or its F(ab')₂ fragment has been coupled beforehand, and, after washing out said solid support, incubating the protease fixed thereto with reagents which allow determination of its activity.

9. The diagnostic method as claimed in claim 8, wherein the antibody directed against the blood clotting factor VII-activating protease (FSAP) is **characterized in that** it is formed by either of the hybridoma cell lines DSM ACC 2453 and 2454.

10. The use of the mutants as claimed in claims 1 to 4 for preparing a medicament for the prophylaxis and/or therapy of bleedings in the case of congenital or acquired deficiency in FVIII, von Willebrand factor, FV, FIX, FX, FXI, FXII and/or antibodies directed against said proteins.

11. A method for preparing monoclonal antibodies to the FSAP mutant as claimed in either of claims 3 and 4, wherein peptides as claimed in claim 5 are used in a method known per se for immunization.

12. A sequence-specific hybridization probe for use in a method known per se for detecting a single nucleotide polymorphism in an FSAP mutant as claimed in either of claims 1 and 2, wherein said single nucleotide polymorphism is a G/A base exchange at nucleotide position 1601, as comprised in SEQ ID No. 2.

## Revendications

1. Mutant dans la séquence ADN codant pour la protéase (FSAP) activant le facteur de coagulation sanguine VII et les activateurs du plasminogène à chaîne unique, **caractérisé en ce que** ledit mutant présente en position 1601 du nucléotide un échange de base G/A par rapport à la séquence nucléotidique selon SEQ ID n° 1.

2. Mutant selon la revendication 1, **caractérisé en ce qu'**il présente, en outre, un échange de base G/C en position 1177 du nucléotide.

3. Mutant de FSAP, **caractérisé en ce que** le mutant présente en position 534 de l'acide aminé un échange gly/glu par rapport à la séquence d'acide aminé selon SEQ ID n° 3.

4. Mutant selon la revendication 4, **caractérisé en ce que** le mutant présente en outre un échange glu/gln en position 393 de l'acide aminé.

5. Protéines ou peptides du mutant selon les revendications 3 à 4, **caractérisés en ce qu'**ils contiennent les acides aminés 534 à 539 de SEQ ID n° 4.

6. Procédé diagnostique in vitro de détection de personnes présentant une expression hétéro zygote ou homozygote, due à la génétique, du mutant FSAP selon les revendications 1 à 4, **caractérisé par une mise en évidence** de l'ADN génomique ou de l' ARNm qui en est déduit.

7. Procédé diagnostique in vitro de détection de personnes présentant une expression hétéro ou homozygote, due à la génétique, du mutant FSAP selon les revendications 1 à 4, **caractérisé par** la mise en évidence de l'ADN génomique, l'ADN génomique étant isolé à partir du sang.

8. Procédé diagnostique in vitro de détection de personnes présentant une expression hétéro ou homozygote, due à la génétique, du mutant FSAP selon les revendications 1 à 4, **caractérisé en ce qu'**on mesure l'activité du mutant FASP en incubant l'échantillon contenant la protéase sur un support solide auquel on a précédemment couplé un anticorps dirigé contre la protéase, ses fragments Fab ou F(ab')₂, et après rinçage du support solide, on incube la protéase qui y est fixée avec des réactifs qui permettent d'en déterminer l'activité.

9. Procédé diagnostique selon la revendication 8, **caractérisé en ce que** l'anticorps dirigé contre la protéase (FASP) activant le facteur de coagulation VII est **caractérisé en ce qu'**il est formé d'une des lignées cellulaires d'hybridomes DSM ACC 2453 ou 2454.

10. Utilisation des mutants selon les revendications 1 à 4 pour la fabrication d'un médicament pour la prophylaxie et/ou la thérapie d'hémorragies en cas de déficit inné ou acquis de FVIII, de facteur de Willebrand FV, FIX, FX, FXI, FXII et/ou d'anticorps dirigés contre ces protéines.

11. Procédé de fabrication d'anticorps monoclonaux contre les mutants FSAP selon la revendication 3 et la revendication4, **caractérisé en ce que**, dans un procédé connu d'immunisation, on utilise des peptides selon la revendication 5.

12. Sonde d'hydridation spécifique à la séquence à utiliser dans un procédé connu pour mettre en évidence un polymorphisme d'un seul nucléotide, dit "single nucleotide polymorphism" dans un mutant FSAP selon l'une quelconque des revendications 1 à 2, **caractérisée en ce que** ledit "single nucleotide polymorphism" inclut un échange de base G/A en position 1601 du nucléotide, comme dans SEQ ID n° 2.
